# FASCICULE DE BREVET EUROPEEN

(11) **EP 3 945 856 B1**
(45) Date de publication et mention de la délivrance du brevet: **05.11.2025**
(21) Numéro de dépôt: 20710166.8
(22) Date de dépôt: 13.03.2020
(51) Int. Cl.: A23P 30/25, A23L 7/109, A23L 19/10, A23L 29/238, A23L 29/244, A23L 29/262, A61P 1/00

(54) **PROCÉDÉ DE PRÉPARATION D'UN SOLIDE ALIMENTAIRE, SOLIDE ALIMENTAIRE COMPRENANT DU GLUCOMMANNANE DE KONJAC ET SON UTILISATION**
VERFAHREN ZUR HERSTELLUNG EINES LEBENSMITTELFESTSTOFFES, LEBENSMITTELFESTSTOFF, DER KONJAC-GLUCOMANNAN ENTHÄLT, UND SEINE VERWENDUNG
PROCESS FOR THE PREPARATION OF A FOOD SOLID, FOOD SOLID COMPRISING KONJAC GLUCOMANNAN AND ITS USE

(30) Priorité: 26.03.2019 FR 1903114
(43) Date de publication de la demande: 09.02.2022
(73) Titulaire: Samain, Daniel, François, Jean, Marie, 31500 Toulouse (FR)
(72) Inventeur: Samain, Daniel, François, Jean, Marie, 31500 Toulouse (FR)
(74) Mandataire: BARRE LAFORGUE
(86) Numéro de dépôt international: PCT/EP2020/056963
(87) Numéro de publication internationale: WO 2020/193238

(56) Documents cités:
- EP-A1- 0 310 703
- EP-A1- 2 668 850
- EP-A1- 3 406 143
- EP-B1- 0 310 703
- WO-A1-03/015538
- WO-A1-2008/111195
- WO-A1-2018/109707
- WO-A1-98/33395
- CN-A- 107 156 643
- CN-A- 107 691 965
- CN-A- 108 294 259
- KR-A- 20180 057 422
- US-A1- 2013 216 662

## Description

L'invention concerne un procédé de préparation d'un solide alimentaire essentiellement non amylacé, un tel solide alimentaire et ses utilisations en alimentation.

Selon l'OCDE (Organisation de Coopération et de Développement Économiques), dans le monde en 2017 un adulte sur deux et un enfant sur six était en surpoids ou était obèse. En France, le taux de personnes obèses atteignait 17% en 2017. Bien que des dispositions soient prises pour combattre le surpoids et l'obésité dans certains pays, la proportion de personnes en surpoids ou obèses est en constante augmentation et les projections de l'OCDE sur les prochaines années suivent la tendance de cette augmentation.

Le surpoids et l'obésité constituent un facteur de risque majeur de survenue de maladies chroniques telles que les maladies cardio-vasculaires et le diabète, en particulier le « diabète de type 2 » ou « diabète non insulinodépendant » (DNID). Le surpoids et l'obésité sont principalement dus à une alimentation hypercalorique déséquilibrée combinée à un déficit d'activité physique et éventuellement à des facteurs génétiques et/ou psychologiques prédisposant au surpoids et/ou à l'obésité. Est représentative d'une telle alimentation hypercalorique déséquilibrée, une alimentation riche en produits amylacés (tels que le pain, les pâtes, les féculents en général) et/ou sucrés (riches en saccharose) conduisant à une augmentation du taux de glucose sanguin, à une activation de la synthèse d'acides gras et du stockage de triglycérides et à une inhibition de la lipolyse. Une telle alimentation hypercalorique est déséquilibrée dans le sens où, par suite de variations importantes de la glycémie qu'elle entraine, elle ne procure pas de sensation durable de satiété, de sorte que le consommateur éprouve une sensation de faim et le besoin de s'alimenter fréquemment pour satisfaire durablement son appétit.

L'invention vise à proposer un solide alimentaire de substitution d'aliments hypercaloriques.

L'invention vise à proposer un tel solide alimentaire de substitution ne conduisant pas à un apport massif de glucose dans l'organisme lors de sa digestion.

En outre, l'excès de glucose sanguin peut conduire au phénomène connu de « glycation » de protéines, de nature à modifier les propriétés de certaines protéines, cette modification pouvant être à l'origine de dysfonctionnements cellulaires, de pathologies et/ou de réactions inflammatoires et/ou auto-immunes. De telles pathologies sont susceptibles d'apparaitre d'autant plus fréquemment chez des personnes âgées, présentant une hyperglycémie chronique (pré-diabète ou diabète) et un catabolisme ralenti de dégradation des produits de « glycation ».

L'invention vise donc à proposer un solide alimentaire apte à limiter la « glycation » de protéines.

Également, une alimentation restreinte à des produits amylacés et pauvres en amidon résistant et/ou sucrés, et rapidement hydrolysés lors de la digestion ne permet pas d'apporter une quantité de fibres solubles et à hydrolyse retardée, nécessaires à un transit digestif harmonieux, mais également nécessaire au maintien et au développement du microbiote formé de la flore intestinale -notamment de la flore microbiologique du colon-.

La consommation de tels produits essentiellement amylacés et/ou sucrés et pauvres en fibres solubles et insolubles ne permet pas de procurer un effet de satiété suffisant et suffisamment durable et de nature à éviter la survenue précoce de la sensation de faim.

L'invention vise à proposer un procédé de préparation d'un solide alimentaire et un tel solide alimentaire à titre de substitut d'aliments amylacés.

De telles solutions passent en général par une consommation d'aliments riches en fibres solubles, non hydrolysable ou peu hydrolysables dans l'estomac et l'intestin grêle, mais hydrolysables par des microorganismes du microbiote du colon.

Au Japon, on connait le « *konnyaku* » obtenu traditionnellement par traitement par l'hydroxyde de calcium d'une farine de tubercules *d'Amorphophallus konjac* (farine de « *konjac »)* à une concentration comprise entre 1 et 3 g/L (0,1 à 0,3% en masse) dans l'eau. Le « *konnyaku »* est utilisé en général sous forme de vermicelles (« *shirataki* »), sous forme de « grains de riz » ou sous forme de pavé. Le « *konnyaku »* est un aliment solide cohésif. Les vermicelles de « *shirataki* » sont non gonflants.

On connait de US 5 173 321 du *« konnyaku »* aromatisé et un procédé de préparation d'un tel *« konnyaku »* aromatisé dans lequel on mélange de la poudre raffinée de « *konnyaku »* et des arômes dans une quantité d'eau à une température comprise entre 20°C et 30°C. La proportion massique de poudre raffinée de « *konnyaku »* dans le mélange est comprise entre 2,3% et 4,5%. Une gélification du mélange est obtenue ultérieurement par addition d'hydroxyde de calcium. Un tel *« konnyaku »* est non gonflant et ne permet pas d'absorber un milieu liquide aromatisé. Il ne permet donc pas une aromatisation extemporanée par simple contact avec un mets liquide aromatisé. Il ne participe pas à la mise en valeur des propriétés organoleptiques d'un mets. Ce type d'aliment est également connu de EP3406143, de WO2008/111195, de CN108294259, de KR20180057422, de CN107691965, de WO98/33395 et de EP0310703.

Cependant, le « *konnyaku »* et le « *shirataki* » n'augmentent pas de volume lors de la digestion et ne permettent pas de procurer une sensation de satiété.

Par ailleurs, certaines formes de *« konnyaku »* sont conditionnées en sachet dans la saumure réactionnelle de sorte que le consommateur occidental est confronté, à l'ouverture du sachet, à une odeur forte et inhabituelle, qu'il associe à un aliment avarié. Il est impératif pour ce consommateur de procéder à de multiples rinçages du *« konnyaku ».*

L'invention vise à pallier des inconvénients de ces formes de *« konnyaku ».*

L'invention vise à proposer un procédé de préparation d'un solide alimentaire et un solide alimentaire doté de propriété de gonflement lors de son parcours gastrique et apte à procurer une sensation de satiété au consommateur, notamment par effet de remplissage de l'estomac.

L'invention vise en particulier à proposer un tel solide alimentaire qui présente au moins l'un des avantages suivants :
- il est hypocalorique, notamment acalorique,
- il ne contribue pas à la glycémie,
- il contribue à l'apport de fibres solubles favorables au développement d'une flore diversifiée du microbiote intestinal.

L'invention vise à proposer un tel solide alimentaire riche en fibres alimentaires.

L'invention vise à proposer un tel solide alimentaire présentant un rapport équilibré entre des fibres solubles et des fibres insolubles.

L'invention vise à proposer un tel solide alimentaire pour son effet régulateur du transit intestinal.

L'invention vise aussi à proposer un tel solide alimentaire riche en fibres solubles dont l'hydrolyse par des microorganismes du microbiote est suffisamment lente pour pouvoir se produire pendant toute la durée de séjour desdites fibres solubles dans le côlon.

L'invention vise aussi à proposer un tel solide alimentaire de substitution d'aliments riches en fibres mais également riches en amidon -tels que des céréales complètes-, et/ou d'aliments riches en fibres mais également riches en sucres -tels que les fruits-.

L'invention vise à proposer un tel solide alimentaire de faible indice glycémique.

L'invention vise à proposer un tel solide alimentaire susceptible d'absorber au moins une partie de sauce d'un mets en sauce.

L'invention vise à proposer un tel solide alimentaire qui soit appétent.

L'invention vise à proposer un procédé de préparation d'un solide alimentaire dans lequel on réalise un mélange :
- d'une quantité d'une farine comprenant au moins un polysaccharide, dit hétéromannane, choisi dans le groupe formé des glucomannanes et des galactomannanes, et
- d'une quantité d'une composition liquide aqueuse,

**caractérisé en ce que** ledit hétéromannane est en quantité telle que le rapport de la masse dudit hétéromannane dans le mélange sur la masse de ladite composition liquide aqueuse dans le mélange est compris entre 5% et 35%, et ;
**en ce qu**'on réalise le mélange par agitation vigoureuse, ce par quoi on forme une dispersion, dite dispersion coulante, sensiblement homogène de la farine dans la composition liquide aqueuse -ladite dispersion coulante comprenant ledit hétéromannane- et de viscosité dynamique inférieure à 100 Pa.s, ladite dispersion coulante évoluant ensuite spontanément pour former un solide cohésif aqueux sensiblement exempt de composition liquide aqueuse libre et de viscosité dynamique supérieure à la viscosité dynamique de ladite dispersion coulante, puis ;
on réalise une étape de maturation du solide cohésif aqueux et de durcissement du solide cohésif aqueux de façon à former le solide alimentaire, le solide alimentaire formé étant non adhésif -notamment non adhésif au toucher- et non coalescent par contact à température d'usage et pression atmosphérique ;
et en ce que ledit hétéromannane comprenant au moins un glucomannane - notamment au moins un glucomannane de tubercule *d'Amorphophallus konjac- ,* on ne fait subir audit hétéromannane aucun traitement par un agent alcalin, notamment aucun traitement par de l'hydroxyde de calcium (Ca(OH)₂) ou par du carbonate de sodium.

Dans tout le texte, le terme « farine » désigne de façon commune une poudre de granulométrie moyenne inférieure à 1 mm, notamment inférieure à 500 µm, de préférence comprise entre 50 µm et 400 µm, plus préférentiellement comprise entre 100 µm et 300 µm.

Dans toute la suite, l'expression « sensiblement homogène » signifie que ladite dispersion coulante étant formée d'une matrice homogène formée de particules de ladite farine dispersées dans ladite composition liquide aqueuse, la matrice est susceptible d'inclure des amas de particules de ladite farine présentant une proportion de composition liquide aqueuse inférieure à la proportion de composition liquide aqueuse de ladite matrice, le rapport de la masse de tels amas sur la masse de ladite dispersion coulante est inférieur à 10% -notamment inférieur à 5%, de préférence inférieur à 1%-. En particulier, ce rapport est inférieur à 4%, notamment inférieur à 3%, de préférence inférieur à 2%, plus préférentiellement inférieur à 0,5%. Encore plus préférentiellement, on réalise ce mélange de façon que ladite dispersion coulante soit exempte d'amas de particules de ladite farine.

Dans tout le texte, la « non-coalescence » désigne la propriété du solide alimentaire selon l'invention par laquelle des parties, particules, pièces ou fragments formé(e)s de ce solide alimentaire ne fusionnent pas spontanément par simple contact à température d'usage du solide alimentaire et pression atmosphérique. On entend par « température d'usage » la température du mets dans lequel le solide alimentaire est destiné à être utilisé.

Dans un procédé selon l'invention, la quantité de composition liquide aqueuse mise en contact de la quantité de farine est inférieure ou égale à la quantité maximale de ladite composition liquide aqueuse susceptible de pouvoir être absorbée intégralement par ladite quantité de farine.

Dans un procédé selon l'invention, on réalise une dispersion rapide, notamment instantanée, de la farine dans la composition liquide aqueuse, ce par quoi on forme ladite dispersion coulante dans laquelle les particules de farine sont réparties à l'état dissocié dans la composition liquide aqueuse, les particules de farine de ladite dispersion aqueuse évoluant spontanément par absorption de la totalité de composition liquide aqueuse disponible pour former un solide cohésif aqueux de viscosité dynamique supérieure à la viscosité dynamique de ladite dispersion coulante.

Dans un procédé selon l'invention, on interrompt l'agitation vigoureuse à l'obtention de ladite dispersion coulante de viscosité dynamique inférieure à 100 Pa.s, et on laisse ladite dispersion coulante évoluer spontanément pour former le solide cohésif aqueux sensiblement de viscosité dynamique supérieure à la viscosité dynamique de ladite dispersion coulante.

On mesure la viscosité dynamique de ladite dispersion coulante et/ou du solide cohésif aqueux par tout moyen connu de l'homme du métier, notamment au moyen d'un viscosimètre rotatif ou d'un viscosimètre vibrant.

L'inventeur suppose que l'étape de mélange par agitation vigoureuse de la farine et de la composition liquide aqueuse permet de conduire à une dispersion homogène des particules de farine dans la composition liquide aqueuse, préalablement à l'hydratation des particules. Cette hydratation, qui s'effectue nécessairement avec une quantité de composition liquide aqueuse restreinte, permettrait une réorganisation des domaines hydrophobes et hydrophiles de ces particules à l'état dispersé, conduisant à la formation du solide cohésif aqueux. **Il** suppose que la formation de ce solide cohésif aqueux procède d'une réorganisation sous contrainte d'au moins certains domaines hydrophobes et d'au moins certains domaines hydrophiles dudit hétéromannane -probablement d'une réorientation de domaines hydrophobes et de domaines hydrophiles des particules dudit hétéromannane-, conduisant à un état métastable du solide cohésif aqueux, de cohésion augmentée par rapport à la cohésion de ladite dispersion coulante et susceptible d'évoluer par maturation -notamment par maturation thermique- vers le solide alimentaire stabilisé, souple, non adhésif, non coalescent et susceptible de pouvoir être mis en forme par extrusion -notamment par extrusion à chaud-. Il suppose que cette dispersion rapide et vigoureuse suivie de l'hydratation des particules de la farine conduit à la formation de particules -notamment de particules dudit hétéromannane- dans lesquelles les domaines hydrophiles se réorganisent, du fait de cette dispersion et de cette hydratation subséquente, vers l'intérieur des particules et les domaines hydrophobes se réorganisent vers l'extérieur des particules en développant des propriétés de cohésion.

L'inventeur a observé que l'étape de maturation conduit à un changement d'aspect du solide cohésif aqueux passant d'un aspect opaque à un aspect translucide. L'inventeur estime que ce changement d'aspect qui s'ajoute aux modifications des propriétés physiques susmentionnées, reflète la disparition des particules de farine hydratée au profit d'objets de plus grandes dimensions.

Dans certains modes de réalisation d'un procédé selon l'invention, lors de l'étape de maturation, on laisse le solide cohésif aqueux au repos pendant une durée supérieure à quelques heures -notamment supérieure à 48 heures- et à température ambiante.

Rien n'empêche cependant que dans d'autres modes de réalisation, on réalise l'étape de maturation par chauffage du solide cohésif aqueux à une température supérieure à 80°C -notamment comprise entre 80°C et 120°C- de façon à former le solide alimentaire. On réalise l'étape de maturation par chauffage du solide cohésif aqueux pendant une durée suffisante pour former le solide alimentaire. L'inventeur suppose que l'étape de maturation permet une stabilisation du solide cohésif aqueux et son durcissement par des interactions hydrophobes établies entre les particules de ladite farine -notamment entre les particules dudit hétéromannane-.

Dans certains modes de réalisation d'un procédé selon l'invention, on réalise une étape de mise en forme du solide alimentaire. On réalise cette étape de mise en forme par extrusion. Dans certains modes particuliers de réalisation d'un procédé selon l'invention, on réalise cette étape de mise en forme par extrusion à chaud du solide alimentaire, notamment à une température comprise entre 80°C et 120°C. Avantageusement, on réalise l'extrusion à chaud du solide alimentaire chaud à l'issue de l'étape de chauffage. Rien n'empêche cependant de dissocier ces deux étapes et de procéder à cette extrusion à chaud dans un extrudeur chauffant, à partir d'un solide alimentaire à température ambiante. On forme un solide alimentaire extrudé, stable, non adhésif et non coalescent.

Par un procédé selon l'invention, on forme d'abord le solide cohésif aqueux qui est souple, c'est-à-dire déformable, sensiblement non élastique en traction et en compression et non adhésif. Par maturation du solide cohésif aqueux, on forme un solide alimentaire durcit, cohésif -c'est-à-dire un solide alimentaire qui est stable sous l'effet des contraintes internes au solide alimentaire-, non adhésif et non coulant. On forme un tel solide alimentaire qui est cohésif et qui, de façon totalement surprenante s'agissant d'un solide alimentaire comprenant au moins un hétéromannane et de l'eau, n'est pas adhésif -notamment sensiblement non adhésif au toucher-.

Dans certains modes de réalisation d'un procédé selon l'invention, ledit au moins un hétéromannane est un glucomannane. Il peut s'agir d'un glucomannane gonflant, apte à s'hydrater, à absorber la composition liquide aqueuse et augmenter sa masse par gonflement.

Avantageusement et selon l'invention, on réalise le mélange par agitation vigoureuse de ladite quantité de farine et de ladite quantité de composition liquide aqueuse, ladite agitation étant maintenue pendant une durée d'au plus 3 minutes, notamment pendant une durée d'au plus 2 minutes, de préférence pendant une durée de l'ordre de 1 minute.

Dans un mode de réalisation particulier d'un procédé selon l'invention, la composition liquide aqueuse est à une température inférieure à +15°C lors du mélange par agitation vigoureuse. On réalise le mélange par agitation vigoureuse, rapide et continue de la quantité de farine et de la quantité de composition liquide aqueuse, la composition liquide aqueuse étant maintenue à une température inférieure à +15°C. Dans ce mode de réalisation particulier, la composition liquide aqueuse est à une température comprise entre +1°C et +10°C, de préférence de l'ordre de +4°C. Dans ce mode de réalisation particulier de mélange à basse température, le rapport de la masse dudit hétéromannane sur la masse de ladite composition liquide aqueuse peut être compris entre 15% à 35%. Rien n'empêche cependant que le rapport de la masse dudit hétéromannane sur la masse de ladite composition liquide aqueuse soit compris entre 5% et 15%.

De façon totalement surprenante et inattendue, l'inventeur a observé que ce mélange à froid conduit à un solide cohésif aqueux, de viscosité dynamique augmentée par rapport à la viscosité de ladite dispersion coulante, sensiblement non adhésif -notamment non adhésif au toucher-, et à un solide alimentaire durci, cohésif, non coulant, non adhésif -notamment non adhésif au toucher- et susceptible de pouvoir être extrudé pour former un solide alimentaire extrudé non-coalescent et sensiblement non élastique. A titre de comparaison uniquement, le mélange réalisé lentement et avec une agitation insuffisamment vigoureuse, ne permet pas d'assurer la dispersion des particules avant leur hydratation. Il se forme un mélange hautement hétérogène et opaque à la lumière, contenant des particules hautement hydratées et des particules faiblement hydratées. Ce mélange est très peu cohésif et se fracture en fragments de petite taille, notamment par extrusion, et ne permet pas une extrusion en filaments continus.

Dans certains modes de réalisation avantageux, on réalise l'étape de maturation du solide cohésif aqueux et de durcissement du solide cohésif aqueux par chauffage. Dans certains modes de réalisation avantageux, on réalise l'étape de maturation du solide cohésif aqueux et de durcissement du solide cohésif aqueux par chauffage du solide cohésif aqueux dans un réacteur clos sous pression autogène. Dans certains modes de réalisation, on chauffe le solide cohésif aqueux dans un autoclave clos à une température de l'ordre de 118°C et pendant une durée suffisante pour permettre le durcissement du solide cohésif aqueux. Dans certains modes de réalisation, on chauffe le solide cohésif aqueux sous pression pendant une durée inférieure à 3 minutes. Le solide alimentaire formé est d'une dureté supérieure à la dureté du solide cohésif aqueux. Il est stable, cohésif, sensiblement non adhésif au toucher, non-coalescent et susceptible de pouvoir être extrudé. Rien n'empêche cependant de réaliser l'étape de maturation à température ambiante et à pression atmosphérique pendant une durée de plusieurs heures ou de plusieurs jours.

Dans certains modes de réalisation, ledit hétéromannane est en quantité massique telle que le rapport de la quantité massique dudit hétéromannane sur la quantité massique de ladite composition liquide aqueuse est compris entre 5% et 30%, notamment compris entre 5% et 25% -en particulier compris entre 7% et 23%, de préférence compris entre 10% et 20%-.

Dans certains modes de réalisation, les quantités de farine et de composition liquide aqueuse dans le mélange étant des quantités en masse, le rapport de la quantité de farine sur la quantité de ladite composition liquide aqueuse est compris entre 5% et 60%, notamment compris entre 5% et 45%. Dans certains modes de réalisation, le rapport de la quantité de farine sur la quantité de ladite composition liquide aqueuse est compris entre 5% et 25%, notamment compris entre 10% et 20%. Dans ces modes de réalisation, les solides alimentaires formés sont avantageusement destinés à la préparation de mets froids. De tels solides alimentaires sont non coalescents à leur température d'usage. Dans d'autres modes de réalisation, le rapport de la quantité de farine sur la quantité de ladite composition liquide aqueuse est compris entre 20% et 60%, notamment compris entre 20% et 40%. Dans ces autres modes de réalisation, les solides alimentaires formés sont avantageusement destinés à la préparation de mets chauds, tels que des soupes chaudes. De tels solides alimentaires sont non coalescents à leur température d'usage, c'est-à-dire à chaud. On adapte la quantité de composition liquide aqueuse et la quantité de farine selon les propriétés organoleptiques souhaitées.

Dans certains modes de réalisation avantageux d'un procédé selon l'invention, la farine comprend une farine de tubercule *d'Amorphophallus konjac.* Dans certains modes de réalisation avantageux selon l'invention, la farine est formée d'une farine de tubercule *d'Amorphophallus konjac.* Dans ces modes de réalisation, ledit hétéromannane est un glucomannane n'ayant subi aucun traitement par un agent alcalin, notamment par de l'hydroxyde de calcium (Ca(OH)₂) ou du carbonate de sodium. Ledit hétéromannane est un glucomannane au moins partiellement acétylés, qui présente la propriété de s'hydrater et dont la masse augmente par mélange avec une composition liquide aqueuse. Dans ces modes de réalisation, le solide cohésif aqueux est sensiblement translucide.

Dans certains modes de réalisation d'un procédé selon l'invention, seul ou en combinaison, ledit au moins un hétéromannane est un galactomannane. Il peut s'agir d'un galactomannane gonflant, apte à s'hydrater, à absorber la composition liquide aqueuse et augmenter sa masse par gonflement. Dans ces modes de réalisation, ledit galactomannane est en quantité massique telle que le rapport de la quantité massique dudit hétéromannane sur la quantité massique de ladite composition liquide aqueuse est compris entre 7% et 35%, notamment compris entre 7% et 30% -en particulier compris entre 10% et 30%, de préférence compris entre 10% et 20%-.

Dans certains modes de réalisation d'un procédé selon l'invention, la farine comprend une quantité d'au moins une fibre insoluble. Par « fibre insoluble » on entend de façon commune, un polymère non digérable par les enzymes digestives -notamment par les amylases- produites dans le tube digestif humain et non digérables par les microorganismes symbiotiques constitutifs du microbiote digestif et/ou intestinal. De telles fibres insolubles non digérées ne contribuent pas à la glycémie et présentent un pouvoir calorique faible. Dans ces modes de réalisation d'un procédé selon l'invention, on choisit au moins une fibre insoluble dans le groupe formé des celluloses, des hémicelluloses, des chitines, du son de blé, du son d'avoine, des lignines, des tannins, notamment. Il est possible également d'utiliser des particules minérales telles que l'hydroxyapatite ou l'argile. Les produits à base de « *konnyaku »* peuvent aussi être broyés et servir de fibres insolubles de même que des polymères modifiés chimiquement par réticulation. Dans certains modes de réalisation avantageux selon l'invention, au moins une fibre insoluble est de la cellulose, notamment de la cellulose microcristalline. Dans ces modes de réalisation d'un procédé selon l'invention, la(les) quantité(s) de fibre(s) insoluble(s) étant une(des) quantité(s) massique(s), le rapport de cette(ces) quantité(s) sur la quantité dudit hétéromannane est compris entre 25% et 75%.

Dans certains modes de réalisation, rien n'empêche que la farine comprenne une quantité d'au moins une fibre soluble distincte dudit hétéromannane. Par « fibre soluble » on entend de façon commune, un oligomère ou un polymère non digérable par les enzymes digestives -notamment par les amylases- produites dans le tube digestif humain, mais digérables par les microorganismes symbiotiques constitutifs du microbiote digestif et/ou intestinal. De telles fibres solubles, digérées dans la partie terminale du tube digestif (colon) ne contribuent pas à la glycémie et présentent un pouvoir calorique faible. Il peut s'agir d'oligosaccharides, notamment d'oligosaccharides du lait, non digérable dans l'intestin grêle. Il peut s'agir d'un disaccharide, tel que le lactose pour des personnes intolérantes au lactose. Il peut s'agir d'oligosaccharides produits par des bactéries -notamment par *Escherichia coli-* recombinantes ou obtenus par modification chimique d'un oligosaccharide. Il peut s'agir d'amidon résistant présent naturellement dans beaucoup de produits amylacés ou qui est préparé par hydrolyse enzymatique partielle d'amidon. Il peut s'agir d'un milieu de fermentation de levures ou de lait fermenté par du « kéfir » ou par des bactéries lactiques thermophiles. Il peut s'agir d'exopolysaccharides bactériens, de protéines glyquées ou de collagène condensé. Dans certains modes de réalisation d'un procédé selon l'invention, ladite au moins une fibre soluble est choisie dans le groupe formé des inulines, des pectines, des carraghénanes et des alginates. Dans ces modes de réalisation, la farine comprend une quantité en masse d'au moins une fibre soluble telle que le rapport de cette quantité sur la quantité dudit hétéromannane est compris entre 25% et 75%.

Dans certains modes de réalisation d'un procédé selon l'invention, la farine présente une granulométrie moyenne inférieure à 500 µm -notamment comprise entre 100 µm et 300 µm.

Dans certains modes de réalisation d'un procédé selon l'invention, la composition liquide aqueuse est de l'eau. Il peut s'agir d'eau pure. Il peut aussi s'agir d'un tampon salin adapté pour ajuster le pH et/ou la salinité de ladite composition liquide aqueuse. En tout état de cause, la composition liquide aqueuse et le solide alimentaire sont exempts de tout agent alcalin -notamment d'hydroxyde de calcium (Ca(OH)₂) et/ou de carbonate de sodium- de libération de groupe acétate du glucomannane. Rien n'empêche, selon certains modes de réalisation d'un procédé selon l'invention, d'ajouter au moins une protéine, un moût de fermentation -notamment un moût de fermentation comprenant des protéines-, au moins une levure alimentaire -notamment *Saccharomyces cerevisiae-* ou au moins une levure diététique à ladite composition liquide aqueuse. Rien n'empêche non plus, selon certains modes de réalisation d'un procédé selon l'invention, d'ajouter une quantité de cellulose à ladite composition liquide aqueuse ou d'utiliser à titre de composition liquide aqueuse, toute préparation aqueuse de cellulose issue d'un processus industriel. On évite ainsi une étape consommatrice d'énergie de déshydratation de la préparation aqueuse de cellulose.

Dans certains modes de réalisation avantageux d'un procédé selon l'invention, on soumet le solide alimentaire à une étape de mise en forme par extrusion
-notamment par extrusion à chaud, en particulier par extrusion sous pression à chaud-. Avantageusement, on réalise l'extrusion du solide alimentaire à travers une filière de mise en forme de pâtes alimentaires (« spaghettis »). Rien n'empêche d'extruder le solide alimentaire de façon à former une forme de nouille, une forme de riz, une forme de lasagne ou une forme de « wrap ». Avantageusement, on réalise ladite extrusion dans un extrudeur bi-vis. Rien n'empêche cependant de réaliser l'extrusion au moyen d'un ustensile ménager et de cuisine du type « hachoir à viande ».

Dans certains modes de réalisation avantageux d'un procédé selon l'invention, l'étape de mise en forme par extrusion est une étape de co-extrusion d'un solide alimentaire par laquelle on forme simultanément une couche de surface extérieure constituée d'un premier solide alimentaire et un cœur sous-jacent constitué d'un deuxième solide alimentaire distinct du premier solide alimentaire. Dans certains modes de réalisation avantageux d'un procédé selon l'invention, le premier solide alimentaire est un solide alimentaire riche en glucomannanes de « *konjak »* et le deuxième solide alimentaire est un solide alimentaire riche en galactomannanes de guar. Dans certains modes de réalisation avantageux d'un procédé selon l'invention, le premier solide alimentaire est formé à partir de farine de « *konjak »* et le deuxième solide alimentaire est un solide alimentaire formé à partir de farine de guar.

Dans certains modes de réalisation avantageux, le procédé selon l'invention comprend une étape de stérilisation du solide alimentaire. On réalise une telle étape de stérilisation par tout moyen adapté pour la préparation d'un solide alimentaire stérile et susceptible de pouvoir être conservé en préservant sensiblement ses propriétés organoleptiques initiales. On réalise une telle étape de stérilisation à chaud d'un solide alimentaire placé dans un récipient clos et hermétique aux microorganismes et aux fluides -notamment aux gaz-.

Dans certains modes de réalisation avantageux, on prépare le solide alimentaire avec un rapport élevé de la quantité de farine sur la quantité de composition liquide aqueuse de façon que le solide alimentaire placé dans un mets liquide aqueux s'hydrate et atteigne un taux de siccité choisi pour ajuster au mieux les propriétés organoleptiques du mets, en particulier dans un délai raisonnable.

L'invention concerne également un solide alimentaire obtenu par un procédé selon l'invention.

L'invention concerne un solide alimentaire comprenant en mélange :
- une quantité d'une farine comprenant au moins un polysaccharide, dit hétéromannane, choisi dans le groupe formé des glucomannanes et des galactomannanes, et
- une quantité d'une composition liquide aqueuse,

caractérisé en ce que ledit hétéromannane est en quantité telle que le rapport de la masse dudit hétéromannane dans le solide alimentaire sur la masse de ladite composition liquide aqueuse dans le solide alimentaire est compris entre 5% et 35%, et en ce que le solide alimentaire est :
   - cohésif,
   - non adhésif -notamment non adhésif au toucher-,
   - apte à absorber une quantité d'un liquide aqueux par mise en contact dudit solide alimentaire et du liquide aqueux, et
   - adapté pour pouvoir être mis en forme par extrusion ;
et en ce qu'au moins un glucomannane étant un glucomannane de tubercule *d'Amorphophallus konjac,* le solide alimentaire est exempt de tout agent alcalin - notamment d'hydroxyde de calcium (Ca(OH)₂) et/ou de carbonate de sodium- de conversion du glucomannane natif de tubercule *d'Amorphophallus konjac* en glucomannane au moins en partie désacétylé.

On évalue par pesée la quantité de liquide aqueux absorbée par le solide alimentaire en immergeant une quantité initiale de solide alimentaire dans un volume de liquide aqueux à la température de 20°C. Après l'immersion, on prélève, on égoutte et on pèse la totalité du solide alimentaire hydraté. On évalue la prise de poids du solide alimentaire et la prise d'eau par le solide alimentaire.

Avantageusement et selon l'invention, le solide alimentaire est sensiblement non élastique. Le solide alimentaire présente des propriétés organoleptiques d'un solide non caoutchouteux.

Le solide alimentaire est non adhésif et non coalescent par contact de parties dudit solide alimentaire à température d'usage et pression atmosphérique.

Le solide alimentaire est adapté pour pouvoir être mis en forme par extrusion.

Dans certains modes de réalisation, un solide alimentaire selon l'invention comprend -notamment majoritairement- au moins un glucomannane -notamment au moins un glucomannane de farine de « *konjak »-* et/ou au moins un galactomannane -notamment au moins un galactomannane de guar-.

Dans certains modes de réalisation, un solide alimentaire selon l'invention comprend au moins une fibre insoluble -notamment de la cellulose-.

Dans certains modes de réalisation, au moins un glucomannane est un glucomannane de tubercule d'*Amorphophallus konjac* et le solide alimentaire est exempt de tout agent alcalin -notamment d'hydroxyde de calcium (Ca(OH)₂) et/ou de carbonate de sodium- de conversion du glucomannane natif de tubercule d'*Amorphophallus konjac* en glucomannane au moins en partie désacétylé.

Dans certains modes de réalisation, le solide alimentaire se présente sous forme de fils non coalescents par simple contact entre eux, en particulier à température d'usage et pression atmosphérique. Le solide alimentaire se présente à l'état fractionné, chaque fraction étant allongée et présentant sensiblement une même forme selon toute section droite transversale (« spaghettis »), les fractions étant non coalescentes entre elles par contact.

Dans certains modes de réalisation, le solide alimentaire selon l'invention est stérile.

Dans certains modes de réalisation, le solide alimentaire selon l'invention est conditionné à l'état stérile dans un récipient clos et hermétique aux microorganismes et aux fluides -notamment aux gaz-. Le solide alimentaire extrudé est non coalescent, en particulier lors de sa stérilisation humide à chaud sous pression.

Le solide alimentaire selon l'invention est exempt de tout agent alcalin -notamment d'hydroxyde de calcium (Ca(OH)₂) et/ou de carbonate de sodium-.

L'invention concerne également un tel solide alimentaire pour son utilisation à titre de médicament. Le solide alimentaire peut être utilisé dans l'alimentation humaine et/ou animale -notamment dans l'alimentation d'animaux de compagnie-.

L'invention concerne aussi un tel solide alimentaire qui peut être utilisé en alimentation -notamment en alimentation humaine et/ou animale-. L'invention concerne aussi un tel solide alimentaire pour son utilisation comme aliment de substitution à une alimentation amylacée.

Le solide alimentaire peut être utilisé dans une étape de confection d'un mets destiné à être consommé froid, notamment à une température inférieure à 25°C. Il peut s'agir d'un « smoothie » à consommer à basse température, notamment comprise entre 1°C et 10°C. Le solide alimentaire est non coalescent à sa température d'usage comprise entre 1°C et 10°C. Il peut s'agir d'un solide alimentaire à consommer en salade à une température de l'ordre de 15°C à 30°C. Le solide alimentaire est non coalescent à sa température d'usage comprise entre 15°C et 30°C.

Le solide alimentaire peut être utilisé dans une étape de confection d'un mets destiné à être consommé chaud, notamment à une température supérieure à 25°C. Il peut s'agir d'un solide alimentaire à consommer en plat chaud à une température comprise entre 40°C et 60°C. Le solide alimentaire est non coalescent à sa température d'usage comprise entre 40°C et 60°C. Il peut s'agir d'un solide alimentaire à consommer dans une soupe chaude à une température comprise entre 60°C et 100°C. Le solide alimentaire est non coalescent à sa température d'usage comprise entre 60°C et 100°C.

Dans certains modes de réalisation d'un procédé selon l'invention, on prépare un solide alimentaire présentant une couche de surface extérieure constituée d'un premier solide alimentaire et un cœur sous-jacent constitué d'un deuxième solide alimentaire distinct du premier solide alimentaire. Dans certains modes de réalisation, on prépare un solide alimentaire présentant une couche de surface extérieure constituée d'un premier solide alimentaire et un cœur sous-jacent constitué d'un deuxième solide alimentaire distinct du premier solide alimentaire, par co-extrusion du premier solide alimentaire de couche de surface extérieure et du deuxième solide alimentaire de cœur sous-jacent, selon deux directions coaxiales. Dans certains modes de réalisation le premier solide alimentaire extérieur est constitué essentiellement d'au moins un glucomannane. Dans certains modes de réalisation le premier solide alimentaire extérieur est constitué essentiellement d'au moins un glucomannane -notamment de farine de *« konjac »* et le deuxième solide alimentaire sous-jacent est constitué essentiellement d'au moins un galactomannane -notamment de farine de guar-. On forme ainsi un solide alimentaire non coalescent, en particulier non coalescent lors de sa stérilisation à chaud. On prépare un tel solide alimentaire par tout moyen, notamment par co-extrusion selon deux directions coaxiales du premier solide alimentaire sous-jacent et du deuxième solide alimentaire extérieur. Rien n'empêche de préparer un solide alimentaire selon l'invention sous forme de particules présentant un cœur sous-jacent interne inscrit à l'intérieur d'une couche de surface extérieure et enveloppé par celle-ci. Rien n'empêche non plus que le solide alimentaire présente au moins une couche d'au moins un solide alimentaire additionnel interposée entre le cœur sous-jacent -notamment le cœur interne- et la couche de surface extérieure.

Un solide alimentaire selon l'invention présente des propriétés d'absorption d'un liquide aqueux, notamment d'eau, et de gonflement compatible avec son utilisation dans des préparations culinaires. En particulier, un tel gonflement lent et retardé d'un solide alimentaire selon l'invention au cours de la digestion permet d'assurer une sensation de satiété au consommateur, limitant la ration alimentaire à la quantité de nourriture nécessaire à son métabolisme. Par sa composition ajustée et équilibrée en fibres insolubles et en fibres solubles, en particulier en fibres solubles du type « hétéromannane » et d'index glycémique réduit, il permet la régulation rhéologique du transit digestif, notamment du transit intestinal, de limiter l'apport massif de glucose lors de la digestion et de favoriser le développement d'une flore microbienne intestinale du microbiote, variée et protectrice vis-à-vis de pathologies métaboliques digestives.

D'autres buts, caractéristiques et avantages de l'invention apparaîtront à la lecture de la description suivante et des exemples donnés à titre non limitatif de certains de ses modes de réalisation possibles de l'invention.

Dans un procédé selon l'invention, on réalise un solide alimentaire à partir d'une farine contenant principalement des polysaccharides choisis parmi des glucomannanes et des galactomannanes et une composition liquide aqueuse par un procédé selon l'invention sans former un solide fortement adhésif et inadapté pour une mise en forme par extrusion. On forme un solide alimentaire non adhésif à partir de farine contenant de la farine de « *konjac »* sans nécessiter de traitement alcalin du mélange de la farine et d'eau par l'hydroxyde de calcium, nécessaire à l'obtention du *« konnyaku ».* Le solide alimentaire selon l'invention ne dégage pas d'odeur désagréable pour un consommateur occidental caractéristique du *« konnyaku ».* L'intérêt alimentaire, culinaire et gastronomique du solide alimentaire selon l'invention découle de ses propriétés organoleptiques ajustables. En outre, le solide alimentaire selon l'invention présente des propriétés de gonflement dans l'eau -quasiment inexistantes dans le *« konnyaku »* qui est stable indéfiniment et ne gonfle pas- lui conférant un pouvoir satiétant supérieur au pouvoir satiétant du « *konnyaku ».* Également le solide alimentaire selon l'invention est formé de fibres solubles digérables par les microorganismes du microbiote et à leur profit, contrairement au « *konnyaku »* dont les polysaccharides modifiés par le traitement alcalin sont peu digérables.

Dans un procédé de préparation d'un solide alimentaire selon l'invention, on choisit ou on confectionne une farine comprenant au moins un polysaccharide choisi dans le groupe formé des glucomannanes et des galactomannanes.

Les glucomannanes sont des polysaccharides de haut poids moléculaires formés d'une chaine principale constituée de D-glucose et de D-mannose liés par des liaisons β-(I-4) et selon une distribution non régulière du D-glucose et du D-mannose. Le rapport de la quantité de D-mannose et de la quantité de D-glucose d'un glucomannane, les quantités étant exprimées en moles, est de l'ordre de 1,6. Certains hydroxyles de D-mannose des glucomannanes de « *konjac »* sont porteurs de groupements acétyles en position C2 ou C3 ou C6. Parmi les glucomannanes, on utilise de préférence les glucomannanes de tubercules d'*Amorphophallus konjac.*

Les galactomannanes sont des polysaccharides formés d'une chaine principale constituée de galactose et de mannose liés par des liaisons β-(I-4). Parmi les galactomannanes, on utilise au moins un galactomannane choisi dans le groupe formé d'un galactomannane de la gomme de Fenugrec dans lequel le rapport du nombre de moles de mannose sur le nombre de moles de galactose est de l'ordre de 1/1, d'un galactomannane de la gomme de guar dans lequel le rapport du nombre de moles de mannose sur le nombre de moles de galactose est de l'ordre de 2/1, d'un galactomannane de la gomme de tara dans lequel le rapport du nombre de moles de mannose sur le nombre de moles de galactose est de l'ordre de 3/1, d'un galactomannane de la gomme de caroube dans lequel le rapport du nombre de moles de mannose sur le nombre de moles de galactose est de l'ordre de 4/1 ou d'un galactomannane de la gomme de casse dans lequel le rapport du nombre de moles de mannose sur le nombre de moles de galactose est de l'ordre de 5/1.

Dans un procédé de préparation d'un solide alimentaire selon l'invention, on choisit ou on confectionne une farine comprenant au moins un galactomannane de guar. Un tel galactomannane permet un apport de galactose assurant la diversification des microorganismes constitutifs du microbiote.

Dans certains modes de réalisation, la farine est un mélange d'une poudre de cellulose -notamment de cellulose microcristalline- et de farine de « *konjac ».* Dans certains modes de réalisation, la cellulose présente une granulométrie moyenne comprise entre 100 µm et 300 µm. Rien n'empêche que la farine comprenne au moins un composé choisi dans le groupe formé d'une inuline, de lignine, de tannins, d'hémicellulose, de collagène, de pectine etc...

Dans un procédé de préparation d'un solide alimentaire selon l'invention, on forme ladite dispersion coulante par mélange -notamment à basse température- et sous contrainte mécanique d'une farine comprenant au moins un polysaccharide choisi dans le groupe formé des glucomannanes et des galactomannanes dans une composition liquide aqueuse. Cette étape de mélange peut être réalisée par apport rapide par saupoudrage de la quantité de farine sous agitation vigoureuse dans la quantité de composition liquide aqueuse -notamment d'eau- froide. On réalise cette dispersion de façon à former transitoirement une dispersion coulante de viscosité dynamique inférieure à 100 Pa.s évoluant spontanément pour former le solide cohésif aqueux sensiblement exempt de composition liquide aqueuse libre et de viscosité dynamique supérieure à la viscosité dynamique de ladite dispersion coulante, puis on réalise la maturation et le durcissement à chaud du solide cohésif aqueux de façon à former le solide alimentaire.

Après l'étape de maturation par chauffage, des pâtes sont extrudées à haute température et sous haute pression au travers de filières d'un extrudeur pour donner des produits de formes et de tailles diverses, nouilles, riz, lasagnes, wrap etc.

Un solide alimentaire selon l'invention permet de limiter la part des glucides (monosaccharides et disaccharides) dans la ration alimentaire. Il permet aussi une libération durable de nutriments au cours de la digestion. Il permet de procurer une sensation de satiété par apport de fibres et par gonflement de ces fibres et évite la tentation d'une surconsommation d'aliments.

Un solide alimentaire selon l'invention constitue un aliment non amylacé susceptible de procurer des sensations organoleptiques de texture et de goût choisies et de satiété par un apport équilibré de fibres solubles et de fibres insolubles. Un tel solide alimentaire permet de tenir compte des spécificités digestives de chaque consommateur, telles que l'âge, le sexe, le niveau d'activité physique, la vitesse de transit digestif, de l'état de santé général et la biodiversité et le dynamisme du microbiote.

Exemple 1 : Farine de « *konjac ».* On prépare un solide alimentaire selon l'invention par ajout rapide et sous agitation vigoureuse d'une masse de farine de tubercules d'*Amorphophallus konjac* de granulométrie de 120 mesh (Kalys Gastronomie, France) dans une masse d'eau à la température de 4°C. Typiquement, pour un volume de 100 mL d'eau à 4°C, on réalise l'ajout de la quantité de farine correspondante en moins de 5 secondes, notamment de l'ordre de 3 secondes. Les quantités de farine et d'eau sont données au tableau 1 ci-après. On forme une dispersion coulante évoluant en un solide cohésif aqueux non adhésif. Une étape de maturation du solide cohésif aqueux et non adhésif est réalisée à une température de 118°C sous pression autogène en autoclave ou en dispositif autocuiseur. On obtient un solide alimentaire cohésif et non adhésif et de dureté augmentée par rapport au solide cohésif aqueux. On soumet ce solide alimentaire durci à une extrusion de façon à donner au solide alimentaire la forme de « spaghettis ». De préférence, on extrude le solide alimentaire présentant un rapport (masse de farine/masse d'eau) supérieur ou égal à 15% à haute température. De préférence, on extrude le solide alimentaire présentant un rapport (masse de farine/masse d'eau) inférieur à 15% à basse température. De préférence, on extrude le solide alimentaire présentant un rapport (masse de farine/masse d'eau) de l'ordre de 5% à une température de l'ordre de +4°C. On obtient un solide alimentaire cohésif, non adhésif sous forme de « spaghettis » sensiblement non coalescents par contact.

On évalue la vitesse de prise d'eau du solide alimentaire selon l'invention. On place une quantité initiale de solide alimentaire dans un volume d'eau en excès à la température de 20°C. 30, 60, 120 et 180 minutes après le mélange de la quantité de solide alimentaire et de la quantité d'eau, on prélève, on égoutte et on pèse la totalité du solide alimentaire. On évalue ainsi la prise de poids du solide alimentaire et la vitesse de prise d'eau par le solide alimentaire. Les résultats sont donnés au tableau 1 ci-après dans lequel la colonne « A » représente la masse (en grammes) de farine apportée pour la formation du solide alimentaire, la colonne « B » représente la masse (en grammes) d'eau apportée pour la formation du solide alimentaire, la colonne « C » représente le rapport de la masse (en grammes) de farine apportée pour la formation du solide alimentaire sur la masse (en grammes) de solide alimentaire, la colonne « D » représente l'augmentation de la masse (en grammes) du solide alimentaire maintenu pendant 30 min dans un excès d'eau à 20°C, la colonne « E » représente l'augmentation de la masse (en grammes) du solide alimentaire maintenu pendant 60 min dans un excès d'eau à 20°C, la colonne « F » représente l'augmentation de la masse (en grammes) du solide alimentaire maintenu pendant 120 min dans un excès d'eau à 20°C et la colonne « G » représente l'augmentation de la masse (en grammes) du solide alimentaire maintenu pendant 180 min dans un excès d'eau à 20°C. Les valeurs entre parenthèses représentent le rapport (en pourcentage) entre la variation de masse et la masse de départ du solide alimentaire.

**[Tableau 1]**

| A | B | C | D | E | F | G |
|---|---|---|---|---|---|---|
| Farine, g | Eau, g | | 30 min | 60 min | 120 min | 180 min |
| 5 | 100 | 4,8% | 12 g (11,4%) | 23 g (21,9%) | / | / |
| 7,5 | 100 | 7,0% | 14 g (13,0%) | 27 g (25,1%) | / | / |
| 10 | 100 | 9,1% | 16 g (14,5%) | 33 g (30,0%) | / | / |
| 15 | 100 | 13,0% | 45 g (39,1%) | 80 g (69,5%) | / | / |
| 25 | 100 | 20,0% | 55 g (44,0%) | 98 g (78,4%) | 150 g (120,0%) | 207 g (165,6%) |
| 40 | 100 | 28,5% | 100 g (71,4%) | 180 g (128,6%) | 205 g (146,4%) | 320 g (228,6%) |

On observe une augmentation de la masse du solide alimentaire plongé dans l'eau pure à 20°C, ladite augmentation étant révélatrice des propriétés de gonflement du solide alimentaire selon l'invention. La cinétique d'augmentation de la masse du solide alimentaire plongé dans un excès d'eau à la température de 20°C est cependant lente, représentative de l'état métastable du matériau constitutif du solide alimentaire et compatible avec son utilisation dans des préparations culinaires. Cette cinétique lente de prise d'eau par le solide alimentaire selon l'invention contraste avec la cinétique excessivement rapide d'hydratation de la farine de « *konjac »* non traitée par un procédé selon l'invention. Les propriétés de non-adhésion et de dureté du solide alimentaire selon l'invention contrastent avec les propriétés adhésives de la farine de *« konjac* » hydratée par addition d'eau à température ambiante. Ces propriétés de non-adhésion et de dureté du solide alimentaire selon l'invention permettent une mise en forme du solide alimentaire, par exemple par extrusion et sa stérilisation à haute température.

On réalise également un essai similaire en plaçant une quantité de solide alimentaire selon l'invention dans un volume d'eau en excès à la température de 100°C. Les résultats sont donnés au tableau 2 ci-après dans lequel la colonne « A » représente la masse (en grammes) de farine apportée pour la formation du solide alimentaire, la colonne « B » représente la masse (en grammes) d'eau apportée pour la formation du solide alimentaire, la colonne « C » représente le rapport de la masse (en grammes) de farine apportées pour la formation du solide alimentaire sur la masse (en grammes) de solide alimentaire, la colonne « H » représente l'augmentation de la masse (en grammes) du solide alimentaire maintenu pendant 30 min dans un excès d'eau à 100°C et, entre parenthèses, le rapport (en pourcentage) entre la variation de masse et la masse de départ du solide alimentaire.

**[Tableau 2]**

| A | B | C | H |
|---|---|---|---|
| Farine, g | Eau, g | | 15 min |
| 10 | 100 | 9,1% | 30 g (28,6%) |
| 15 | 100 | 13,0% | 80 g (74,4%) |
| 25 | 100 | 20,0% | 128 g (116,4%) |
| 40 | 100 | 28,5% | 213 g (185,2%) |

La cinétique d'augmentation de la masse du solide alimentaire plongé dans un excès d'eau à la température de 100°C est cependant lente, représentative de l'état métastable du matériau constitutif du solide alimentaire et compatible avec son utilisation dans des préparations culinaires.

Les solides alimentaires décrits à l'exemple 1 sont stables lors de leur stérilisation ultérieure. À partir d'une valeur de 20% du rapport de la masse (en grammes) de farine apportée pour la formation du solide alimentaire sur la masse (en grammes) de solide alimentaire et pour des valeurs de ce rapport supérieures à 20%, il n'est pas observé de coalescence des « spaghettis » lors de leur stérilisation à chaud. En particulier, les « spaghettis » se dissocient par simple immersion dans l'eau. Rien n'empêche cependant de préparer le solide alimentaire extemporanément, notamment sous la forme de « spaghettis », en vue de son utilisation dans une préparation culinaire.

Les solides alimentaires décrits à l'exemple 1 formés à partir de farine de tubercules d'*Amorphophallus konjac* contiennent essentiellement des glucomannanes. Dès lors que la partie supérieure du tube digestif humain, formée de la bouche, du pharynx, de l'œsophage, de l'estomac et de l'intestin grêle, n'est pas pourvue d'une composition enzymatique adaptée à la digestion de glucomannanes, les solides alimentaires décrits à l'exemple 1 atteignent le colon sans avoir été hydrolysés, sans avoir libéré de glucose, ne contribuent pas de façon significative à la glycémie et présentent un pouvoir calorique de l'ordre de 2 Kcal par gramme de glucomannane. Ils sont hydrolysés par les microorganismes du colon qui produisent des enzymes adaptées pour cette hydrolyse, en libérant du glucose et du mannose utilisables par les microorganismes du colon pour leur propre métabolisme et le maintien de leur diversité.

Il est à noter que les aliments amylacés raffinés tels que le pain blanc et le riz blanc, sont hydrolysés et réabsorbés avant d'atteindre l'intestin grêle et ne contribuent pas au maintien et au développement de microorganismes du microbiote. Il résulte d'une consommation exclusive de tels aliments amylacés raffinés, un dépérissement d'au moins une partie de ces microorganismes et un appauvrissement du microbiote.

Exemple 2 : Farine de « guar ». On prépare un solide alimentaire selon l'invention par un procédé tel que décrit à l'exemple 1 par ajout rapide et sous agitation vigoureuse d'une masse de farine de Guar (Kalys Gastronomie, France) dans une masse d'eau à la température de 4°C. Typiquement, pour un volume de 100 mL d'eau à 4°C, on réalise l'ajout de la quantité de farine correspondante en moins de 5 secondes, notamment de l'ordre de 3 secondes. Les quantités de farine et d'eau sont données au tableau 3 ci-après, dans lequel la colonne « I » représente la masse (en grammes) de farine de « guar » apportée pour la formation du solide alimentaire, la colonne « J » représente la masse (en grammes) d'eau apportée pour la formation du solide alimentaire, la colonne « K » représente le rapport de la masse (en grammes) de farine apportée pour la formation du solide alimentaire sur la masse (en grammes) de solide alimentaire. Le solide alimentaire formé à partir de farine de guar selon un procédé conforme au procédé décrit à l'exemple 1 est cohésif et non adhésif et de dureté augmentée par rapport au solide alimentaire formé à l'issue du mélange à 4°C. On soumet ce solide alimentaire durci à une extrusion de façon à donner au solide alimentaire la forme de « spaghettis ». On évalue comme décrit à l'exemple 1, la vitesse de prise d'eau du solide alimentaire extrudé selon l'invention. Au tableau 3, les colonnes « L », « M » et « N » représentent l'augmentation de la masse (en grammes) du solide alimentaire maintenu pendant 60 min, 120 min et 210 min respectivement, dans un excès d'eau à 20°C et, entre parenthèses, le rapport (en pourcentage) entre la variation de masse et la masse de départ du solide alimentaire.

**[Tableau 3]**

| I | J | K | L | M | N |
|---|---|---|---|---|---|
| Farine, g | Eau, g | | 60 min | 120 min | 210 min |
| 10 | 100 | 9,1% | 35 g (31,8%) | / | / |
| 15 | 100 | 13,0% | 80 g (69,6%) | / | / |
| 25 | 100 | 20,0% | 108 g (86,4%) | 160 g (128,0%) | 172,8 g (120,0%) |
| 40 | 100 | 28,6% | 134 g (95,7%) | 217 g (155,0%) | 311 g (222,1%) |

On observe une augmentation de la masse du solide alimentaire plongé dans l'eau pure à 20°C, ladite augmentation étant révélatrice des propriétés de gonflement du solide alimentaire selon l'invention. La cinétique d'augmentation de la masse du solide alimentaire plongé dans un excès d'eau à la température de 20°C est cependant lente, représentative de l'état métastable du matériau constitutif du solide alimentaire et compatible avec son utilisation dans des préparations culinaires.

Les solides alimentaires décrits à l'exemple 2 sont stables lors de leur stérilisation ultérieure. A partir d'une valeur de 25% du rapport de la masse (en grammes) de farine apportée pour la formation du solide alimentaire sur la masse (en grammes) de solide alimentaire et pour des valeurs de ce rapport supérieures à 25%, il n'est pas observé de coalescence des « spaghettis » lors de leur stérilisation à chaud et de leur conservation sous forme stérilisée à chaud.

Rien n'empêche cependant de préparer le solide alimentaire, notamment sous la forme de « spaghettis », extemporanément et sans stérilisation en vue de son utilisation dans une préparation culinaire. On utilise avantageusement pour une extrusion à froid, un dispositif du type « presse-purée à levier » communément utilisé en cuisine. Rien n'empêche non plus de conserver le solide alimentaire à basse température, notamment à une température de congélation, en vue de son utilisation ultérieure dans une préparation culinaire.

Exemple 3 : Farine mixte de « *konjac »* et de « guar ». On prépare un solide alimentaire selon l'invention par un procédé tel que décrit à l'exemple 1 par ajout rapide et sous agitation vigoureuse d'une masse d'un mélange de farine de tubercules d'*Amorphophallus konjak « konjak »* (Kalys Gastronomie, France) et de farine de Guar (Kalys Gastronomie, France) dans une masse d'eau à la température de 4°C. Typiquement, pour un volume de 100 mL d'eau à 4°C, on réalise l'ajout de la quantité de farine correspondante en moins de 5 secondes, notamment de l'ordre de 3 secondes. Les quantités de farine et d'eau sont données au tableau 4 ci-après dans lequel la colonne « N » représente la masse (en grammes) de farine de « *konjac* » apportée pour la formation du solide alimentaire, la colonne « O » représente la masse (en grammes) de farine de « guar » apportée pour la formation du solide alimentaire, la colonne « P » représente la masse (en grammes) d'eau apportée pour la formation du solide alimentaire, la colonne « Q » représente le rapport de la masse (en grammes) de farine apportées pour la formation du solide alimentaire sur la masse (en grammes) de solide alimentaire. Le solide alimentaire formé à partir de farine mixte de « *konjac »* et de « guar » selon un procédé conforme au procédé décrit à l'exemple 1 est cohésif et non adhésif et de dureté augmentée par rapport au solide alimentaire formé à l'issue du mélange à 4°C. On soumet ce solide alimentaire durci à une extrusion de façon à donner au solide alimentaire la forme de « spaghettis » au moyen d'un dispositif presse-purée à levier.

On évalue la vitesse de prise d'eau du solide alimentaire extrudé selon l'invention comme décrit à l'exemple 1. Au tableau 4, les colonnes « R » et « S » décrivent l'augmentation de la masse (en grammes) du solide alimentaire maintenu pendant 60 min et 120 min respectivement dans un excès d'eau à 20°C et, entre parenthèses, le rapport (en pourcentage) entre la variation de masse et la masse de départ du solide alimentaire.

**[Tableau 4]**

| N | O | P | Q | R | S |
|---|---|---|---|---|---|
| Farine de *« konjac »,* g | Farine de « guar », g | Eau, g | | 60 min | 120 min |
| 20 | 20 | 100 | 28,6% | 177 g (126,4%) | 233 g (166,4%) |

On observe une augmentation de la masse du solide alimentaire plongé dans l'eau pure à 20°C, ladite augmentation étant révélatrice des propriétés de gonflement du solide alimentaire selon l'invention. La cinétique d'augmentation de la masse du solide alimentaire plongé dans un excès d'eau à la température de 20°C est cependant lente, représentative de l'état métastable du matériau constitutif du solide alimentaire et compatible avec son utilisation dans des préparations culinaires.

Exemple 4 : Farine mixte de « *konjac »* et de fibre insoluble, la cellulose. On prépare un solide alimentaire selon l'invention par un procédé tel que décrit à l'exemple 1 par ajout rapide et sous agitation vigoureuse d'une masse d'un mélange de farine de tubercules d'*Amorphophallus konjak « konjak »* (Kalys Gastronomie, France) et de cellulose dans une masse d'eau à la température de 4°C. Typiquement, pour un volume de 100 mL d'eau à 4°C, on réalise l'ajout de la quantité de farine correspondante en moins de 5 secondes, notamment de l'ordre de 3 secondes. Les quantités de farine et d'eau sont données au tableau 5 ci-après dans lequel la colonne « T » représente la masse (en grammes) de farine de « *konjac* » apportée dans la farine pour la formation du solide alimentaire, la colonne « U » représente la masse (en grammes) de cellulose microcristalline apportée dans la farine pour la formation du solide alimentaire, la colonne « V » représente la masse (en grammes) d'eau apportée pour la formation du solide alimentaire, la colonne « W » représente le rapport de la masse (en grammes) de farine apportées pour la formation du solide alimentaire sur la masse (en grammes) de solide alimentaire.

Le solide alimentaire formé à partir de farine mixte de « *konjac »* et de cellulose selon un procédé conforme au procédé décrit à l'exemple 1 est cohésif et non adhésif et de dureté augmentée par rapport au solide alimentaire formé à l'issue du mélange à 4°C. On soumet ce solide alimentaire durci à une extrusion de façon à donner au solide alimentaire la forme de « spaghettis » au moyen d'un dispositif presse-purée à levier.

On évalue la vitesse de prise d'eau du solide alimentaire extrudé selon l'invention comme décrit à l'exemple 1. Au tableau 5, les colonnes « X » et « Y » décrivent l'augmentation de la masse (en grammes) du solide alimentaire maintenu pendant 60 min et 120 min respectivement dans un excès d'eau à 20°C et, entre parenthèses, le rapport (en pourcentage) entre la variation de masse et la masse de départ du solide alimentaire.

**[Tableau 5]**

| T | U | V | W | X | Y |
|---|---|---|---|---|---|
| Farine de *« konjac »,* g | Cellulose, g | Eau, g | | 60 min | 120 min |
| 20 | 20 | 100 | 28,6% | 109 g (77,9%) | 144 g (102,8%) |

On observe une augmentation de la masse du solide alimentaire plongé dans l'eau pure à 20°C, ladite augmentation étant révélatrice des propriétés de gonflement du solide alimentaire selon l'invention. La cinétique d'augmentation de la masse du solide alimentaire plongé dans un excès d'eau à la température de 20°C est cependant lente, représentative de l'état métastable du matériau constitutif du solide alimentaire et compatible avec son utilisation dans des préparations culinaires. Il a été observé que l'utilisation d'une farine formée par mélange de farine de « *konjac »* et de cellulose microcristalline permet de former un solide alimentaire de stabilité augmentée en comparaison avec le solide alimentaire obtenu avec la seule farine de « *konjac ».* Il a été également observé que l'utilisation de farine de *« konjac* » masque le goût désagréable de la cellulose, notamment pour une valeur du rapport de la masse de farine de *« konjac »* sur la masse de cellulose supérieure ou égale à 25% et pour une proportion de farine de « *konjac »* dans le solide alimentaire supérieure ou égale à 9% (rapport de la masse de farine de *« konjac »* sur la masse de solide alimentaire supérieur ou égal à 10%).

Exemple 5 : Solide alimentaire mixte élaboré à partir d'une farine mixte de *« konjac »* et de cellulose. On prépare un solide alimentaire selon l'invention par un procédé tel que décrit à l'exemple 1, dans lequel la farine est une farine mixte comprenant des proportions massiques de 50% de farine de « *konjac »* et de 50% de cellulose. On mélange rapidement 30 g de ladite farine mixte dans 100 mL d'eau à la température de +4°C sous agitation vigoureuse. On obtient un solide alimentaire cohésif, non adhésif et sensiblement non coalescent et dans lequel le goût désagréable de la cellulose est masqué.

Exemple 6 : Solide alimentaire mixte élaboré à partir d'une farine mixte de *« konjac »* et de cellulose. On prépare un solide alimentaire selon l'invention par un procédé tel que décrit à l'exemple 1 mais dans lequel la farine est une farine mixte comprenant des proportions massiques de 1/3 de farine de « *konjac »* et de 2/3 de cellulose. On mélange 30 g de ladite farine mixte dans 100 mL d'eau à la température de +4°C. On obtient un solide alimentaire cohésif, non adhésif et sensiblement non coalescent et dans lequel le goût désagréable de la cellulose est masqué.

Exemple 7 : Solide alimentaire mixte élaboré à partir d'une farine mixte de *« konjac »* et de cellulose. On prépare un solide alimentaire selon l'invention par un procédé tel que décrit à l'exemple 1 mais dans lequel la farine est une farine mixte comprenant des proportions massiques de 50% de farine de « *konjac »* et de 50% de cellulose. On mélange 40 g de ladite farine mixte dans 100 mL d'eau à la température de +4°C. On obtient un solide alimentaire cohésif, non adhésif et sensiblement non coalescent et dans lequel le goût désagréable de la cellulose est masqué.

Exemple 8 : Solide alimentaire mixte élaboré à partir d'une farine mixte de *« konjac »* et de cellulose. On prépare un solide alimentaire selon l'invention par un procédé tel que décrit à l'exemple 1 mais dans lequel la farine est une farine mixte comprenant des proportions massiques de 25% de farine de « *konjac »* et de 75% de cellulose. On mélange 40 g de ladite farine mixte dans 100 mL d'eau à la température de +4°C. On obtient un solide alimentaire cohésif, non adhésif et sensiblement non coalescent et dans lequel le goût désagréable de la cellulose est masqué.

Exemple 9 : Solide alimentaire mixte élaboré à partir d'une farine mixte de *« konjac »,* de cellulose, d'inuline, de tara et de pectine. On prépare un solide alimentaire selon l'invention par un procédé tel que décrit à l'exemple 1 mais dans lequel la farine est une farine mixte comprenant de l'ordre de 33% de farine de « *konjac »,* de l'ordre de 33% de cellulose, de l'ordre de 11% de poudre d'inuline, de l'ordre de 11% de poudre de tara et de l'ordre de 11% de poudre de pectine. On mélange 45 g de ladite farine mixte dans 100 mL d'eau à la température de +4°C. On obtient un solide alimentaire cohésif, non adhésif et sensiblement non coalescent et dans lequel le goût désagréable de la cellulose est masqué.

Exemple 10 : Solide alimentaire mixte élaboré à partir d'une farine mixte *« konjac »* et de cellulose. On prépare un solide alimentaire selon l'invention par un procédé tel que décrit à l'exemple 1 mais dans lequel la farine est une farine mixte comprenant 50% de farine de « *konjac »* et 50% de cellulose. On mélange 30 g de ladite farine mixte dans une suspension comprenant 15 g de levure diététique dans 100 mL d'eau à la température de +4°C. On obtient un solide alimentaire cohésif, non adhésif et sensiblement non coalescent et dans lequel les goûts inappropriés et désagréables de la cellulose et de la levure diététique sont masqués. Rien n'empêche d'utiliser un tel solide alimentaire de masquage du goût de tout autre ingrédient ou condiment.

Les solides alimentaires extrudés décrits aux exemples 1 à 10 présentent des propriétés organoleptiques compatibles avec leur utilisation dans une préparation culinaire. Les solides alimentaires selon l'invention sont riches en hétéromannanes, notamment en glucomannanes et/ou en galactomannanes et permettent de diminuer l'impact glycémique de ces solides alimentaires par rapport à l'impact glycémique de compositions essentiellement amylacées.

Un solide alimentaire selon l'invention est équilibré en fibres solubles et en fibres insolubles. Il est rhéofluidifiant et permet un transit digestif rhéologiquement contrôlé. Il forme du fait de la digestion un matériau visqueux, adhésif et hydrophile propice au développement et au maintien des microorganismes du microbiote.

Exemple 11 : Préparation d'un mets alimentaire. On prépare un solide alimentaire selon l'invention par un procédé tel que décrit à l'exemple 1 dans lequel la farine est une farine de « *konjac ».* Par extrusion du solide cohésif aqueux à travers une filière, on obtient un solide alimentaire cohésif, sous forme de filaments ou « spaghettis » non adhésif et sensiblement non coalescent. On mélange le solide alimentaire extrudé avec une sauce à base de tomate. L'inventeur a observé une diffusion de la sauce tomate dans les filaments du solide alimentaire, formant une enveloppe périphérique s'opposant à la coalescence.

D'autres applications sont envisageables dans le domaine alimentaire

## Revendications

1. Procédé de préparation d'un solide alimentaire dans lequel on réalise un mélange :
- d'une quantité d'une farine comprenant au moins un polysaccharide, dit hétéromannane, choisi dans le groupe formé des glucomannanes et des galactomannanes, et
- d'une quantité d'une composition liquide aqueuse,
**caractérisé en ce que** ledit hétéromannane est en quantité telle que le rapport de la masse dudit hétéromannane dans le mélange sur la masse de ladite composition liquide aqueuse dans le mélange est compris entre 5% et 35%, et ;
**en ce qu'**on réalise le mélange par agitation vigoureuse, ce par quoi on forme une dispersion, dite dispersion coulante, sensiblement homogène de la farine dans la composition liquide aqueuse et de viscosité dynamique inférieure à 100 Pa.s, ladite dispersion coulante évoluant ensuite spontanément pour former un solide cohésif aqueux sensiblement exempt de composition liquide aqueuse libre et de viscosité dynamique supérieure à la viscosité dynamique de ladite dispersion coulante, puis ;
on réalise une étape de maturation du solide cohésif aqueux et de durcissement du solide cohésif aqueux de façon à former le solide alimentaire, le solide alimentaire formé étant non adhésif et non coalescent par contact à température d'usage et pression atmosphérique ;
**et en ce que** ledit hétéromannane comprenant au moins un glucomannane, on ne fait subir audit hétéromannane aucun traitement par un agent alcalin, notamment aucun traitement par de l'hydroxyde de calcium (Ca(OH)₂) ou par du carbonate de sodium.

2. Procédé selon la revendication 1, **caractérisé en ce que** la composition liquide aqueuse est à une température inférieure à +15°C lors du mélange par agitation vigoureuse.

3. Procédé selon l'une des revendications 1 ou 2, **caractérisé en ce que** la farine comprend une farine de tubercule d'*Amorphophallus konjac.*

4. Procédé selon l'une des revendications 1 à 3, **caractérisé en ce que** la farine comprend une quantité d'au moins une fibre insoluble.

5. Procédé selon la revendication 4, **caractérisé en ce que** la farine comprend de la cellulose à titre de fibre insoluble.

6. Procédé selon l'une des revendications 1 à 5, **caractérisé en ce que** la farine présente une granulométrie moyenne inférieure à 500 µm.

7. Procédé selon l'une des revendications 1 à 6, **caractérisé en ce que** la composition liquide aqueuse est de l'eau.

8. Procédé selon l'une des revendications 1 à 7, **caractérisé en ce qu'**on soumet le solide alimentaire à une étape de mise en forme par extrusion à travers une filière de mise en forme de spaghettis alimentaires.

9. Procédé selon l'une des revendications 1 à 8, **caractérisé en ce qu'**on prépare un solide alimentaire présentant une couche de surface extérieure constituée d'un premier solide alimentaire et un cœur sous-jacent constitué d'un deuxième solide alimentaire distinct du premier solide alimentaire, par co-extrusion du premier solide alimentaire de couche de surface extérieure et du deuxième solide alimentaire de cœur sous-jacent selon deux directions coaxiales.

10. Procédé selon l'une des revendications 1 à 9, **caractérisé en ce qu'**il comprend une étape de stérilisation du solide alimentaire.

11. Solide alimentaire comprenant en mélange :
- une quantité d'une farine comprenant au moins un polysaccharide, dit hétéromannane, choisi dans le groupe formé des glucomannanes et des galactomannanes, et
- une quantité d'une composition liquide aqueuse,
**caractérisé en ce que** ledit hétéromannane est en quantité telle que le rapport de la masse dudit hétéromannane dans le solide alimentaire sur la masse de ladite composition liquide aqueuse dans le solide alimentaire est compris entre 5% et 35%, et **en ce que** le solide alimentaire est :
- cohésif,
- non adhésif,
- apte à absorber une quantité d'un liquide aqueux par mise en contact dudit solide alimentaire et du liquide aqueux, et ;
- adapté pour pouvoir être mis en forme par extrusion ;
et **en ce que**, au moins un glucomannane étant un glucomannane de tubercule d'*Amorphophallus konjac,* le solide alimentaire est exempt de tout agent alcalin de conversion d'un glucomannane natif de tubercule d'*Amorphophallus konjac* en glucomannane au moins en partie désacétylé.

12. Solide alimentaire selon la revendication 11, **caractérisé en ce qu'**il se présente sous forme de fils non coalescents par simple contact entre eux à température d'usage et pression atmosphérique.

13. Solide alimentaire selon l'une des revendications 11 ou 12, pour son utilisation à titre de médicament.

14. Utilisation d'un solide alimentaire selon l'une des revendications 11 ou 12 en alimentation.

## Patentansprüche

1. Verfahren zur Herstellung eines Lebensmittelfeststoffes, wobei ein Gemisch erstellt wird:
- einer Menge eines Mehls, umfassend mindestens ein Polysaccharid, bezeichnet als Heteromannan, ausgewählt aus der Gruppe, gebildet aus Glucomannnanen und Galactomannanen, und
- einer Menge einer wässrigen flüssigen Zusammensetzung,
**dadurch gekennzeichnet, dass** das Heteromannan eine derartige Menge umfasst, dass das Verhältnis der Masse des Heteromannans in dem Gemisch zur Masse der wässrigen flüssigen Zusammensetzung in dem Gemisch im Bereich zwischen 5 % und 35 % liegt, und:
dadurch, dass die Mischung durch kräftiges Rühren erstellt wird, wodurch eine Dispersion gebildet wird, bezeichnet als flüssige Dispersion, im Wesentlichen homogen des Mehls in der wässrigen flüssigen Zusammensetzung, und mit einer dynamischen Viskosität von weniger als 100 Pa.s, wobei die flüssige Dispersion sich in der Folge spontan entwickelt, um einen wässrigen bindenden Feststoff zu bilden, der im Wesentlichen keine freie wässrige flüssige Zusammensetzung aufweist und eine dynamische Viskosität von mehr als der dynamischen Viskosität der flüssigen Dispersion aufweist, dann:
ein Schritt des Reifens des wässrigen bindenden Feststoffs und des Aushärtens des wässrigen flüssigen Feststoffs durchgeführt wird, um den Lebensmittelfeststoff zu bilden, wobei der gebildete Lebensmittelfeststoff nicht haftend ist und durch Kontakt bei Verwendungstemperatur und atmosphärischem Druck nicht verschmelzen kann;
und dadurch, dass das Heteromannan mindestens ein Glucomannan umfasst, wird das Heteromannan keiner Behandlung durch ein alkalisches Mittel unterzogen, insbesondere keiner Behandlung durch Kalziumhydroxyd (Ca(OH)₂) oder durch Natriumcarbonat.

2. Verfahren nach Anspruch 1, **dadurch gekennzeichnet, dass** die wässrige flüssige Zusammensetzung eine Temperatur von weniger als +15 °C bei der Mischung durch kräftiges Rühren aufweist.

3. Verfahren nach einem der Ansprüche 1 oder 2, **dadurch gekennzeichnet, dass** das Mehl ein Mehl der Knolle von Amorphophallus konjac umfasst.

4. Verfahren nach einem der Ansprüche 1 bis 3, **dadurch gekennzeichnet, dass** das Mehl eine Menge mindestens einer unlöslichen Faser umfasst

5. Verfahren nach Anspruch 4, **dadurch gekennzeichnet, dass** das Mehl Zellulose als unlösliche Faser umfasst.

6. Verfahren nach einem der Ansprüche 1 bis 5, **dadurch gekennzeichnet, dass** das Mehl eine mittlere Granulometrie von weniger als 500 µm aufweist.

7. Verfahren nach einem der Ansprüche 1 bis 6, **dadurch gekennzeichnet, dass** die wässrige flüssige Zusammensetzung Wasser ist.

8. Verfahren nach einem der Ansprüche 1 bis 7, **dadurch gekennzeichnet, dass** der Lebensmittelfeststoff einem Schritt des Formgebens durch Extrusion über eine Spinndüse zur Formgebung von Lebensmittelspaghetti unterzogen wird.

9. Verfahren nach einem der Ansprüche 1 bis 8, **dadurch gekennzeichnet, dass** ein Lebensmittelfeststoff, der eine äußere Oberflächenschicht aufweist, die aus einem ersten Lebensmittelfeststoff und einem darunterliegenden Kern besteht, der aus einem zweiten Lebensmittelfeststoff besteht, der verschieden vom ersten Lebensmittelfeststoff ist, durch Koextrusion des ersten Lebensmittelfeststoffs der äußeren Oberflächenschicht und des zweiten Lebensmittelfeststoffs des darunterliegenden Kerns gemäß zwei koaxialen Richtungen hergestellt wird.

10. Verfahren nach einem der Ansprüche 1 bis 9, **dadurch gekennzeichnet, dass** es einen Schritt der Sterilisierens des Lebensmittelfestoffs umfasst.

11. Lebensmittelfeststoff, umfassend im Gemisch:
- einer Menge eines Mehls, umfassend mindestens ein Polysaccharid, bezeichnet als Heteromannan, ausgewählt aus der Gruppe, gebildet aus Glucomannnanen und Galactomannanen, und
- einer Menge einer wässrigen flüssigen Zusammensetzung,
**dadurch gekennzeichnet, dass** das Heteromannan eine derartige Menge umfasst, dass das Verhältnis der Masse des Heteromannans in dem Lebensmittelfeststoff zu der Masse der wässrigen flüssigen Zusammensetzung in dem Lebensmittelfeststoff im Bereich zwischen 5 % und 35 % liegt, und dadurch, dass der Lebensmittelfeststoff wie folgt ist:
- bindend,
- nicht haftend,
- dazu ausgelegt, eine Menge einer wässrigen Flüssigkeit zu absorbieren durch In-Kontakt-Bringen des Lebensmittelfeststoffs mit der wässrigen Flüssigkeit, und
- dazu angepasst, durch Extrusion in Form gebracht werden zu können;
und dadurch, dass mindestens ein Glucomannan, ein Glucomannan der Knolle von Amorphophallus konjac ist, der Lebensmittelfeststoff frei von jedem alkalischen Mittel zur Umwandlung eines nativen Glucomannans der Knolle von Amorphophallus konjacin ein mindestens teilweise deacetyliertes Glucomannan ist.

12. Lebensmittelfeststoff nach Anspruch 11, **dadurch gekennzeichnet, dass** er die Form von Fäden aufweist, die durch einfachen Kontakt untereinander bei Verwendungstemperatur und atmosphärischem Druck nicht verschmelzend ist.

13. Lebensmittelfeststoff nach einem der Ansprüche 11 oder 12 für seine Verwendung als Medikament.

14. Verwendung eines Lebensmittelfeststoffs nach einem der Ansprüche 11 oder 12 als Lebensmittel.

## Claims

1. Process for preparing a food solid, in which is prepared a mixture:
- of an amount of a flour comprising at least one polysaccharide, named heteromannan, chosen from the group formed from glucomannans and galactomannans, and
- of an amount of an aqueous liquid composition,
**characterized in that** said heteromannan is in an amount such that the ratio of the mass of said heteromannan in the mixture to the mass of said aqueous liquid composition in the mixture is between 5% and 35%, and
**in that** the mixture is prepared by vigorous stirring, via which a substantially homogeneous dispersion is formed, named pourable dispersion, of the flour in the aqueous liquid composition, and having a dynamic viscosity of less than 100 Pa.s, said pourable dispersion then spontaneously changing to form an aqueous cohesive solid which is substantially free of free aqueous liquid composition and which has a dynamic viscosity greater than the dynamic viscosity of said pourable dispersion, and then
a step of maturation of the aqueous cohesive solid and of hardening of the aqueous cohesive solid is performed so as to form the food solid, the formed food solid being non-adhesive and non-coalescent by contact at the temperature of use and atmospheric pressure;
**and in that,** since said heteromannan comprises at least one glucomannan, said heteromannan is not subjected to any treatment with an alkaline agent, notably to any treatment with calcium hydroxide (Ca(OH)₂) or with sodium carbonate.

2. Process according to Claim 1, **characterized in that** the aqueous liquid composition is at a temperature below +15°C during the mixing by vigorous stirring.

3. Process according to either of Claims 1 and 2, **characterized in that** the flour comprises an *Amorphophallus konjac* tuber flour.

4. Process according to one of Claims 1 to 3, **characterized in that** the flour comprises an amount of at least one insoluble fibre.

5. Process according to Claim 4, **characterized in that** the flour comprises cellulose as insoluble fibre.

6. Process according to one of Claims 1 to 5, **characterized in that** the flour has a mean particle size of less than 500 µm.

7. Process according to one of Claims 1 to 6, **characterized in that** the aqueous liquid composition is water.

8. Process according to one of Claims 1 to 7, **characterized in that** the food solid is subjected to a step of forming into shape by extrusion through a die for forming food spaghettis.

9. Process according to one of Claims 1 to 8, **characterized in that** a food solid having an outer surface layer consisting of a first food solid and a subjacent core consisting of a second food solid different from the first food solid is prepared by coextrusion of the first food solid of outer surface layer and of the second food solid of subjacent core, in two coaxial directions.

10. Process according to one of Claims 1 to 9, **characterized in that** it comprises a step of sterilization of the food solid.

11. Food solid comprising, as a mixture:
- an amount of a flour comprising at least one polysaccharide, named heteromannan, chosen from the group formed from glucomannans and galactomannans, and
- an amount of an aqueous liquid composition,
**characterized in that** said heteromannan is in an amount such that the ratio of the mass of said heteromannan in the food solid to the mass of said aqueous liquid composition in the food solid is between 5% and 35%, and **in that** the food solid is:
- cohesive,
- non-adhesive,
- capable of absorbing an amount of an aqueous liquid by placing said food solid and the aqueous liquid in contact, and
- suitable for being formed into shape by extrusion;
and **in that**, since at least one glucomannan is a glucomannan from *Amorphophallus konjac* tuber, the food solid is free of any alkaline agent for conversion of a native *Amorphophallus konjac* tuber glucomannan into at least partly deacetylated glucomannan.

12. Food solid according to Claim 11, **characterized in that** it is in the form of filaments that are not coalescent by simple contact with each other at the temperature of use and atmospheric pressure.

13. Food solid according to either of Claims 11 and 12, for its use as a medicament.

14. Use of a food solid according to either of Claims 11 and 12 in food. ] ]
